# EUROPÄISCHE PATENTSCHRIFT

(11) **EP 1 768 585 B1**
(45) Veröffentlichungstag und Bekanntmachung des Hinweises auf die Patenterteilung: **04.01.2012**
(21) Anmeldenummer: 04738083.7
(22) Anmeldetag: 12.07.2004
(51) Int. Cl.: A61B 17/70

(54) **VORRICHTUNG ZUR DYNAMISCHEN FIXIERUNG VON KNOCHEN**
DEVICE FOR THE DYNAMIC FIXATION OF BONES
DISPOSITIF DE FIXATION DYNAMIQUE D'OS

(43) Veröffentlichungstag der Anmeldung: 04.04.2007
(73) Patentinhaber: Synthes GmbH, 4436 Oberdorf (CH)
(72) Erfinder: SCHLÄPFER, Fridolin, CH-4434 Hölstein (CH)
(74) Vertreter: Lusuardi, Werther
(86) Internationale Anmeldenummer: PCT/CH2004/000443
(87) Internationale Veröffentlichungsnummer: WO 2006/005198

(56) Entgegenhaltungen:
- EP-A- 0 669 109
- EP-A- 0 836 835
- EP-A- 1 210 914
- WO-A-94/00066
- US-A- 5 057 111
- US-A- 6 022 350
- US-A- 6 063 090

## Beschreibung

Die Erfindung bezieht sich auf ein Zwischenelement für eine lösbar blockierbare, kugelgelenkartige Verbindung gemäss dem Oberbegriff des Patentanspruchs 1, sowie auf eine Vorrichtung zur dynamischen Fixierung von Knochen gemäss dem Oberbegriff des Patentanspruchs 1.

Die vorliegende Erfindung betrifft eine Vorrichtung zur dynamischen Stabilisierung von Knochen oder Knochenfragmenten, insbesondere Wirbeln, und umfasst mindestens einen über mindestens zwei Knochenfixationsmittel mit den Wirbelkörpern verbundene Längsträger. Die Knochenfixationsmittel sind vorzugsweise von posterior durch die Pedikel in die Wirbelkörper eingebrachte Pedikelschrauben.

Hauptindikatoren für eine dynamische, insbesondere von posterior durchgeführte Fixation sind ein alters- und/oder krankheitsbedingter Verfall (Degeneration) der Integrität der Wirbelsäulenstruktur, Entzündungen und/oder Verletzungen im Bereich der Bandscheibe, das Bandapparates, der Fazettengelenke und/oder des subchondralen Knochens.

Das Ziel der Erfindung besteht in der Eigenschaft der Verbindung zwischen Längsträger und Knochenfixationsmittel, dass das die Verbindung bildende Verbindungsteil im Bereich seiner Verbindung mit dem Knochenfixationsmittel ein Zwischenelement aufweist, das entweder zusammen mit dem Verbindungsteil oder zusammen mit dem Kopfsegment des Knochenfixationsmittels eine Verbindung bildet, die im unverspannten Zustand eine kugelgelenkartige Bewegung zulässt und im verspannten Zustand einen spannungsarmen, flexiblen Formschluss bildet. Diese Eigenschaften werden bestimmt durch die Geometrie und durch das Material des Zwischenelementes.

Fixationsvorrichungen wie sie in den Patenten WO 94/00066 (Schläpfer und Hess), WO 98/25534 (Schläpfer) und WO 98/52482 (Schläpfer und Hess) beschrieben werden, werden auf Grund ihrer Steifigkeit für eine Fusionierung von Wirbelsegmenten eingesetzt.

Aus der EP-A 0 836 835 RICHELSOPH ist eine Wirbelsäulenfixationsvorrichtung gemäss dem Oberbegriff des Anspruchs 1 bekannt.

Eine Wirbelsäulenfixationsvorrichtung mit einem oder mehreren Knochenfixationsmitteln, einem Längsträger und einem Verbindungsteil ist aus der EP-A 1 210 914 MARQUEZ bekannt.

Eine weitere Wirbelsäulenfixationsvorrichtung mit einem oder mehreren Knochenfixationsmitteln, einem Längsträger und einem Verbindungsteil ist aus der WO 94/00066 SCHLÄPFER bekannt.

Eine Knochenfixationsvorrichtung mit einem longitudinalen Verbindungsteil und mehreren im Verbindungsteil kugelgelenkartig gelagerten und fixierbaren Knochenfixationsmitteln ist aus der US 6,022,350 GANEM bekannt.

Aus der WO 94/00066 SCHLÄPFER ist eine weitere Wirbelsäulenfixationsvorrichtung mit einem oder mehreren Knochenfixationsmitteln, einem Längsträger und einem Verbindungsteil bekannt.

Bei den obengenannten Vorrichtungen wird das Knochenfixationsmittel über ein dazwischen geschaltetes Verbindungselement mit dem Längsträger starr verbunden. Die Verbindung zwischen dem Knochenfixationsmittel und dem Längsträger ist kugelgelenkartig ausgebildet und kann in beliebiger Position blockiert werden. Dadurch ist eine anatomisch gerechte Anpassung der Fixationsvorrichtung gegeben.

Mit der steifen Verbindung sind die Voraussetzungen für eine knöcherne Fusion der überbrücken Wirbelsäulensegment geschaffen, nicht aber für eine mit dem dynamischen Fixationskonzept angestrebte Konvertierung des die Beschwerden bewirkenden Bewegungs- und Belastungsmusters in ein die Beschwerden reduzierendes, den Metabolismus der betroffenen Strukturen förderndes Bewegungs- und Belastungsmusters.

Eine Vorrichtung zur elastisch gedämpften Verbindung zwischen den Kopfsegrnenten von Pedikelschrauben und einem Längsträger ist aus der US 5,480,401 NAVAS bekannt. Diese bekannte Vorrichtung umfasst elastische Spannelemente für die Fixierung der sphärischen Kopfsegmente der Pedikelschrauben innerhalb der Stabilisationsvorrichtung. Nachteilig an dieser bekannten Vorrichtung ist, dass das Kopfsegment reibschlüssig zwischen den elastischen Spannelementen eingespannt ist, so dass das Kopfsegment bei entsprechenden hohen Drehmomenten zwischen dem Knochenfixationsmittel und dem Längsträger verrutschen kann.

Hier will die Erfindung Abhilfe schaffen. Der Erfindung liegt die Aufgabe zugrunde, eine Vorrichtung zur dynamischen Fixation von beeinträchtigten Wirbelsäulensegmenten zu schaffen, welche bezüglich mindestens einer von drei senkrecht zueinander stehenden Achsen eine formschlüssige und Dämpfungseigenschaften aufweisende Verbindung zwischen dem Knochenfixationsmittel und dem Längsträger gestattet.

Die Erfindung löst die gestellte Aufgabe mit einer Vorrichtung zur dynamischen Fixation von Knochen, welche die Merkmale des Anspruchs 1 aufweist.

Die durch die Erfindung erreichten Vorteile sind im wesentlichen darin zu sehen, dass dank der erfindungsgemässen Vorrichtung
- die kugelgelenkartige Verbindung in einer beliebigen Stellung formschlüssig blockierbar ist, indem die Strukturierung am Kopfsegment oder an der Hohlraumwand in die Wand des Gegenstückes gedrückt wird;
- die Verbindung zwischen dem Knochenfixationsmittel und dem Längsträger trotz der formschlüssigen Blockierung des Kopfsegmentes im Zwischenelement die gewünschten Dämpfungseigenschaften aufweist;
- keine Gleitbewegung zwischen den Gelenkteilen stattfindet und die Dynamisierung rein auf der elastischen Deformation des Zwischenelementes basiert; und
- eine kontrollierte Steifigkeit auf Schub und Rotation, d.h. bei Bewegungen, welche bei degenerativen Veränderungen in den Wirbelsegmenten zur Beschwerden und Schmerzen führen können, erreichbar ist.

Die im Anspruch 1 beschriebene, durch das Verbindungsteil gebildete Verbindung zwischen dem Längsträger und dem Knochenfixationsmittel kann in eine Verbindung zwischen Längsträger und Verbindungsteil und eine Verbindung zwischen Verbindungsteil und Knochenfixationsmittel aufgeteilt werden.

Die Verbindung zwischen Verbindungsteil und dem Längsträger ist monoaxial ausgebildet, d.h. diese Verbindung erlaubt im nichtverspannten Zustand eine Längsverschiebung des Verbindungsteils entlang und eine Rotation um den Längsträger.

Die Verbindung zwischen dem Verbindungsteil und dem Knochenfixationsmittel lässt im unverspannten Zustand eine kugelgelenkartige Bewegung zwischen dem Knochenfixationsmittel und dem Verbindungsteil zu und bildet im verspannten Zustand einen Formschluss.

Neben den Spannmitteln zur Verspannung der Verbindungen zwischen Verbindungsteil und Längsträger sowie Verbindungsteil und Knochenfixationsmittel enthält das Verbindungsteil ein das Verbindungsteil und das Knochenfixationsmittel separierendes Zwischenelement.

Im Gegensatz zu starren Verbindungen für die Fusion von Wrbelkörpern, wo für eine optimale Blockierung der kugelgelenkartigen Verbindung das Zwischenelement mit Vorteil aus einem im Vergleich zum Knochenfixationsmittel und dem Verbindungsteil 20 - 50% weicherem Material hergestellt wird, wird bei einer dynamischen Fixationsvorrichtung das Zwischenelement vorzugsweise aus einem viskoelastischen Material gefertigt. Das Verbindungsteil selbst und das Knochenfixationsmittel besteht mit Vorteil aus dem gleichen harten Material wie bei den starren Verbindungen.

Die im unverspannten Zustand kugelgelenkartige Bewegung zwischen dem Verbindungsteil und dem Knochenfixationsmittel wird entweder durch das besagte Zwischenelement und das Kopfsegment des Knochenfixationsmittels oder durch das besagte Zwischenelement und das Verbindungsteil selbst gebildet. Dabei muss mindestens eine der beiden, die kugelgelenkartige Bewegung bestimmenden, komplementären Zonen sphärisch konvex bzw. sphärisch konkav ausgebildet sein.

Beim Verspannen der im unverspannten Zustand kugelgelenkartigen Verbindung wird ein Formschluss erreicht, wenn die Zone des viskoelastischen Zwischenelementes sphärisch konvex (Kombination Zwischenelement und Verbindungsteil) bzw. sphärisch konkav (Kombination Zwischenelement und Kopfsegment des Knochenfixationsmittels) und die entsprechende komplementäre Zone der im nichtverspannten Zustand kugelgelenkartigen Verbindung strukturiert (z.B. verzahnt) oder asphärisch (z.B. rotationssymmetrisch) ausgebildet ist. Im letztgenannten Fall müssen die beiden komplementären Zonen der im unverspannten Zustand kugelgelenkförmigen Verbindung über mindestens drei räumlich verteilte Punkte im Kontakt sein.

Im Fall einer Strukturierung der zum Zwischenelement komplementären Zone der im unverspannten Zustand kugelgelenkartigen Verbindung entsteht beim Verspannen ein Formschluss indem das weiche Material des Zwischenelementes in die Strukturierung hineingedrückt wird.

Ein mechanisch verbesserter Formschluss entsteht, wenn die zum Zwischenelement komplementäre Zone der kugelgelenkartigen Verbindung asphärisch ausgebildet ist. In diesem Fall passt sich die im unverspannten Zustand sphärische Geometrie des Zwischenelementes der asphärischen Geometrie der komplementären Zone an. Wenn zum Beispiel das Zwischenelement kugelförmig und das Verbindungselement im Kontaktbereich zylindrisch ausgebildet ist, wird beim Verspannen die Kugel in der zylinderischen Bohrung partiell zylindrisch.

Beim Verspannen entstehen im Zwischenelement grosse innere Spannungen. Auf Grund dieser inneren Spannungen verhält sich das Zwischenelement steif. Wenn das Material des Zwischenelementes zumindest partiell viskoelastisch ist, bauen sich die beim Verspannen erzeugten inneren Spannungen über die Zeit ab, ohne dass der Formschluss verloren geht, vorausgesetzt dass es genügend Freiraum gibt, in den das viskoelastische Material fliessen kann. Mit abnehmenden inneren Spannungen nimmt die für das dynamische Verhalten der Fixationsvorrichtung wichtige Flexibilität der Verbindung zu.

Das Zwischenelement ist aus einem viskoelastischen Material, beispielsweise Polycarbonat-Urethan gefertigt. Je nach Viskosität dieses visko-elastischen Materials findet über die Zeit eine mehr oder minder rasche Reduktion der bei der Blockierung des Kugelgelenkes in der Verbindung aufgetretenen Spannungen statt, indem das unter Druck stehende visko-elastische Material in im Design speziell eingeplante, den Formschluss unterstützenden Freiräume kriechen kann. Damit ist der Vorteil erreichbar, dass durch den Abbau der bei der Blockierung des Kugelgelenkes entstandenen internen Spannungen die in situ kreierte formschlüssige Verbindung an Festigkeit und an Elastizität gewinnt.

Wenn das Zwischenelement zumindest partiell aus viskoelastischem Material aufgebaut ist, können mit der Viskosität des Materials die Dämpfungseigenschaften und das elastische Verhalten der kugelgelenkartigen Verbindung beeinflusst werden. Je grösser die Vskosität des Materials umso elastischer verhält sich die kugelgelenkartige Verbindung bei im Alltag auftretenden Kurzzeitbelastungen und um so kleiner ist die Dämpfungskomponente.

Ideal ist ein eher elastisches Verhalten bei im Alltag auftretenden Kurzzeitbelastungen und ein eher viskoses Verhalten auf Langzeit zum Abbauen der internen Spannungen für eine optimale Ausnutzung der Flexibilität des Materials.

Die dreidimensionale makroskopische Strukturierung kann zum Beispiel wie folgt realisiert sein:
- durch konzentrisch zur Zentralachse des Knochenfixationsmittels mindestens partiell ringförmige Erhebungen, welche vorzugsweise peripher spitzig ausgebildet sind;
- durch eine auf dem konvexen oder dem konkaven Gelenkteil mindestens partiell peripher umlaufenden Nute;
- durch mehrere auf dem konvexen oder dem konkaven Gelenkteil mindestens partiell peripher umlaufenden Nuten;
- durch eine auf dem konvexen oder dem konkaven Gelenkteil mindestens partiell peripher umlaufende Abflachung;
- durch eine mindestens partiell zylindrische Ausbildung der zum Zwischenelement komplementären Zone der kugelgelenkartigen Verbindung, wobei im Fall der Gelenkkombination Zwischenelement und Verbindungsteil die zum Zwischenelement komplementäre Zone mindestens partiell die Form eines Hohlzylinders hat;
- durch pyramiden- oder kegelförmige Erhebungen auf der konvexen oder der konkaven Kontaktzone;
- durch eine Kreuzverzahnung, bzw. Rändelung der konvexen oder der konkaven Kontaktzone.

In weiteren Ausführungsformen weisen die Erhebungen eine Höhe zwischen 0,5 mm und 1,5 mm, vorzugsweise zwischen 0,8 mm und 1,2 mm. Damit ist der Vorteil erreichbar, dass die plastische Verformung, d.h. das Kriechen des Wandmaterials im Bereich der Kontaktzone A so optimiert werden kann, dass einerseits ein möglichst voluminöser Formschluss zwischen den aktiven Gelenkteilen erreichbar ist und andererseits die Relaxation des Materials ausreichend ist.

Die Verspannung des Zwischenelementes kann wie folgt realisiert sein:
- durch eine mittels der Spannmittel verkeilbare Konusverbindung zwischen der Aussenwand des Zwischenelementes und der Wand der Kavität im Verbindungsteil, wobei die Spannmittel direkt oder mittels eines Zwischenstückes und/oder des Längsträgers indirekt auf das Zwischenelement drücken können; oder
- durch eine mittels der Spannmittel verkeilbare Konusverbindung zwischen der Oberfläche des Kopfsegmentes des Knochenfixationsmittels und der Hohlraumwand des im Zwischenelement angeordneten Hohlraumes, wobei die Spannmittel direkt oder indirekt auf das Zwischenelement drücken.

Als Spannmittel können Schrauben oder Muttern, welche mit dem Verbindungsteil oder dem Kopfsegment verbindbar sind, eingesetzt werden.

Der Weg des Spannmittels beim Verspannen der Vorrichtung wird vorzugsweise mechanisch auf eine Verspannungsdistanz S begrenzt, so dass die Verspannung kontrolliert werden kann.

Weitere vorteilhafte Ausgestaltungen der Erfindung sind in den abhängigen Ansprüchen gekennzeichnet.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Schnitt durch ein Verbindungsteil zwischen einer Pedikelschraube oder einem Pedikelhaken und einem Längsträger und nicht Teil der Erfindung;
Fig. 2 einen Schnitt durch ein Verbindungsteil zwischen einer Pedikelschraube und einem Längsträger und nicht Teil der Erfindung;
Fig. 3 einen Schnitt durch ein Verbindungsteil zwischen einer Pedikelschraube und einem Längsträger und nicht Teil der Erfindung;
Fig. 4 einen Schnitt durch die in Fig. 3 dargestellte Fixationsvorrichtung im verspannten Zustand;
Fig. 5 einen Schnitt durch eine
Fixationsvorrichtung und nicht Teil der Erfindung; und
Fig. 6 einen Ausschnitt gemäss der Markierung A in Fig. 5;
Fig. 7 einen Ausschnitt gemäss der Markierung A in Fig. 5;
Fig. 8 einen Ausschnitt gemäss der Markierung A in Fig. 5;
Fig. 9 einen Ausschnitt gemäss der Markierung A in Fig. 5;
Fig. 10 einen Ausschnitt gemäss der Markierung A in Fig. 5;
Fig. 11 einen Ausschnitt gemäss der Markierung B in Fig. 4;
Fig. 12 einen Ausschnitt gemäss der Markierung B in Fig. 4;
Fig. 13 einen Ausschnitt gemäss der Markierung B in Fig. 4;
Fig. 14 einen Ausschnitt gemäss der Markierung B in Fig. 4;
Fig. 15 einen Ausschnitt gemäss der Markierung B in Fig. 4 einer anderen Ausführungsform der erfindungsgemässen Fixationsvorrichtung im unverspannten Zustand;
Fig. 16 einen Ausschnitt der in Fig. 15 dargestellten Ausführungsform der erfindungsgemässen Fixationsvorrichtung im verspannten Zustand;
Fig. 17 ein Knochenfixationsmittel und nicht Teil der Erfindung;
Fig. 18 ein Knochenfixationsmittel; und nicht Teil der Erfindung;
Fig. 19 ein Knochenfixationsmittel und nicht Teil der Erfindung;
Fig. 20 ein Knochenfixationsmittel und nicht Teil der Erfindung;
Fig. 21 ein Knochenfixationsmittel und nicht Teil der Erfindung;
Fig. 22 ein Knochenfixationsmittel und nicht Teil der Erfindung;
Fig. 23 eine Ausgestaltung der Kavität im Verbindungsteil und nicht Teil der Erfindung;
Fig. 24 eine Ausgestaltung der Kavität im Verbindungsteil und nicht Teil der Erfindung;
Fig. 25 eine Ausgestaltung der Kavität im Verbindungsteil wiederum einer anderen Ausführungsform der erfindungsgemässen Vorrichtung;
Fig. 26 eine Ausgestaltung der Kavität im Verbindungsteil einer weiteren Ausführungsform der erfindungsgemässen Vorrichtung;
Fig. 27 eine Ausgestaltung der Kavität im Verbindungsteil wiederum einer weiteren Ausführungsform der erfindungsgemässen Vorrichtung; und
Fig. 28 eine Ausgestaltung der Kavität im Verbindungsteil einer anderen Ausführungsform der erfindungsgemässen Vorrichtung.

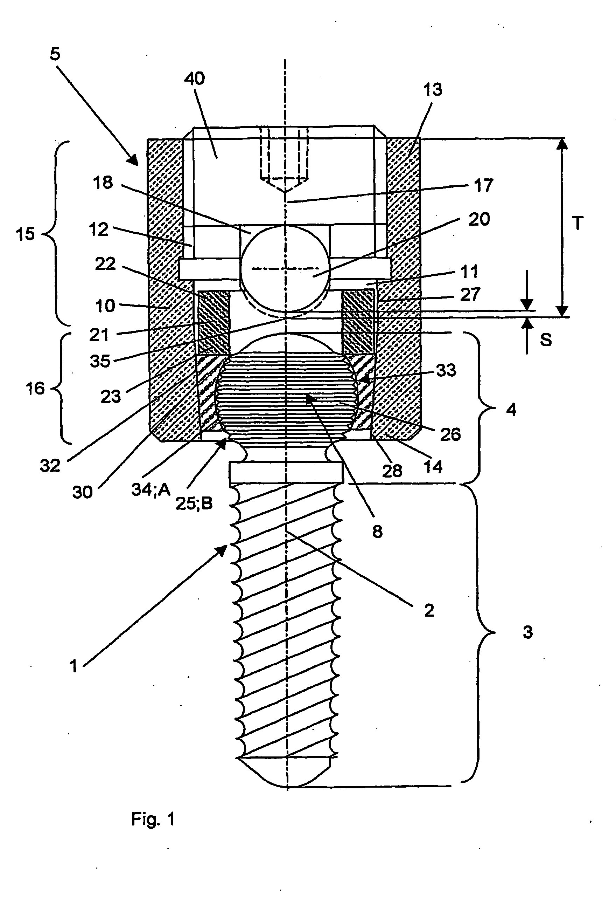

In Fig. 1 ist eine Ausführungsform dargestellt, welche ein als Pedikelschraube ausgebildetes Knochenfixationsmittel 1 mit einer Zentralachse 2 und ein als Hülse 10 ausgebildetes Verbindungsteil 5 zur Verbindung des Knochenfixationsmittels 1 mit einem Längsträger 20 umfasst. Das Knochenfixationsmittel 1 besteht hier aus einem koaxialen, als Schraubenschaft ausgestalteten Verankerungssegment 3 und einem oben an den Schraubenschaft angrenzenden, ebenfalls koaxial angeordneten Kopfsegment 4. Der Längsträger 20 wird vor seiner Fixierung im Verbindungsstück 5 in einen im Verbindungsteil 5 angeordneten Kanal 18 eingelegt und anschliessend mittels der Spannmittel 40 im Kanal 18 fixiert. Der Kanal 18 durchdringt das Verbindungsteil 5 quer zur Zentralachse 2 und ist am oberen Ende 13 des Verbindungsteils 5 offen. Das Kugelgelenk 8 zwischen dem Verbindungsteil 5 und dem Knochenfixationsmittel 1 wird hier durch das Zwischenelement 30 und das Kopfsegment 4 gebildet, wobei die Hohlraumwand 34 des Hohlraumes 33 im Zwischenelement die konkave erste Kontaktzone A bildet und die äussere Oberfläche des Kopfsegmentes 4 die konvexe zweite Kontaktzone B bildet.

Das Kopfsegment 4 des Knochenfixationsmittels 1 ist hier sphärisch ausgebildet und mit einer makroskopischen dreidimensionalen Strukturierung 25 versehen. In der hier dargestellten Ausführungsform ist die makroskopische Strukturierung 25 durch zur Zentralachse 2 des Knochenfixationsmittels 1 konzentrisch angeordnete, ringförmige Erhebungen 26 realisiert. Die ringförmigen Erhebungen 26 sind im Querschnitt dreieckförmig ausgebildet, so dass sich die scharfen Kanten der Erhebungen 26 in das viskoelastischen Material des Zwischenelementes 30 eingraben können.

Das Verbindungsteil 5 umfasst eine Längsachse 17, ein die Längsachse 17 schneidendes, oberes Ende 13, ein die Längsachse 17 schneidendes, unteres Ende 14 und eine Kavität 11, welche hier das Verbindungsteil 5 koaxial vom oberen Ende 13 bis zum unteren Ende 14 durchdringt. Die Kavität 11 besteht aus zwei axial hintereinander angeordneten Segmenten 15;16, wovon das obere Segment 15 eine koaxial zylindrische Bohrung umfasst, in welcher das radial elastisch deformierbare Zwischenelement 30 axial verschiebbar gelagert ist, während das untere Segment 16 sich gegen des untere Ende 14 verjüngend konisch ausgebildet ist. Die Aussenwand 32 des Zwischenelementes 30 ist zum Innenkonus 28 im unteren Segmentes 16 komplementär konisch ausgestaltet, so dass das Zwischenelement 30 - wenn es in der Kavität 11 koaxial gegen das untere Ende 14 des Verbindungsteiles 5 gepresst wird - radial komprimiert wird. Ferner umfasst das Zwischenelement 30 einen axial durchgehend offenen Hohlraum 33, welcher hier zum Kopfsegment 4 des Knochenfixationsmittels 1 komplementär sphärisch ausgebildet ist. Das Kopfsegment 4 ist im dekomprimierten Zustand des Zwischenelementes 30 vom unteren Ende 14 des Verbindungsteiles 5 in den Hohlraum 33 einschnappbar. Im komprimierten Zustand des Zwischenelementes 30 wird das Kopfsegment 4 im Hohlraum 33 blockiert. Wegen des sphärischen Ausgestaltung des Kopfsegmentes 4 und des Hohlraumes 33 ist das Knochenfixationsmittel 1 relativ zum Verbindungsteil 5 polyaxial schwenkbar und auch unter verschiedenen Winkeln zwischen der Längsachse 2 des Knochenfixationsmittels 1 und der Längsachse 17 des Verbindungsteiles 5 blockierbar.

Während der radialen Kompression des Zwischenelementes 30 werden die Erhebungen 26 der makroskopischen Strukturierung 25 am Kopfsegment 4 des Knochenfixationsmittels 1 in die Hohlraumwand 34 des Hohlraumes 33 eingepresst. Das Material des Zwischenelementes 30 ist so gewählt, dass es unter den während der radialen Kompression auftretenden Kräften zu fliessen beginnt und die Hohlraumwand 34 des Hohlraumes 33 komplementär zu der makroskopischen Strukturierung 25 plastisch verformt wird. Dadurch ist ein Formschluss zwischen dem Kopfsegment 4 des Knöchenfixätionsmittels 1 und dem Zwischenelement 30 erreichbar.

Die Vorrichtung ist hier im nicht verspannten Zustand dargestellt, so dass das Kopfsegment 4 noch frei drehbar im Hohlraum 33 des Zwischenelementes 30 gelagert ist. Mittels der Spannmittel 40 kann das Zwischenelement 30 auf einer Verspannungsdistanz S solange verspannt werden, bis der Längsträger 20 am unteren Ende 35 des Kanals 18 aufliegt. Beim Verspannen wird das viskoelastische Material des Zwischenelementes 30 in die dreidimensionale Strukturierung 25 gedrückt. Nach dem Anspannen erfolgt im Zwischenelement 30 ein Spannungsabbau über Kaltfluss.

Die axiale Verschiebung des Zwischenelementes 30 erfolgt hier mittels der als Spannschraube ausgestalteten Spannmittel 40, welche in ein zu ihrem Gewinde komplementäres Innengewinde 12 im oberen Segment 15 der Kavität 11 einschraubbar ist. Das Spannmittel 40 drückt beim Anziehen auf den in den Kanal 18 eingelegten Längsträger 20. Damit beim Anziehen der Spannmittel 40 neben dem Längsträger 20 auch das Kopfsegment 4 des Knochenfixationsmittels 1 im Verbindungsteil 5 fixierbar ist, ist zwischen dem Längsträger 20 und dem Zwischenelement 30 ein ringförmiges Zwischenstück 21 angeordnet. Die Tiefe T des Kanals 18 so gross gewählt, dass das in den Kanal 18 eingelegte Längsträger 20 auf des obere Ende 22 des Zwischenstückes 21 drückt. Das untere Ende 23 des Zwischenstückes 21 liegt auf dem Zwischenelement 30 auf. Beim Anziehen der Spannmittel 40 drückt dieses auf den Längsträger 20, welcher zusammen mit dem angrenzenden Zwischenstück 21 und dem an das Zwischenstück 21 angrenzenden Zwischenelement 30 gegen das untere Ende 14 des Verbindungsteiles 5 gepresst wird. Durch das konisch ausgebildete untere Segment 16 des Verbindungsteils 5 wird das konische Zwischenelement 30 radial komprimiert und das Kopfsegment 4 des Knochenfixationsmittels 1 im Hohlraum 33 des Zwischenelementes 30 blockiert.

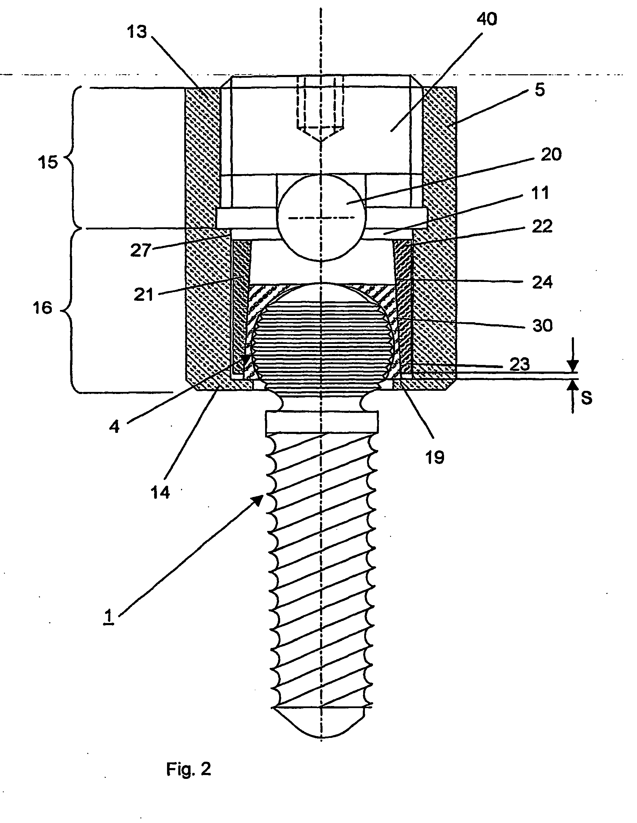

Die in Fig. 2 dargestellte Ausführungsform unterscheidet sich von der in Fig. 1 dargestellten Ausführungsform nur darin, dass das untere Segment 16 der Kavität 11 im Verbindungsteil 5 kreiszylindrisch ausgebildet ist, und das Zwischenstück 21 einen sich vom oberen Ende 22 des Zwischenstückes 21 zum unteren Ende 23 des Zwischenstücks 21 erweiternden Innenkonus 24 aufweist, und das Zwischenelement 30 aussen, sich gegen das obere Ende 13 des Verbindungsteils 5 verjüngend komplementär konisch ausgebildet ist, so dass beim Anziehen der Spannmittel 40 dieses wiederum auf den Längsträger 20 drückt, welcher zusammen mit dem angrenzenden Zwischenstück 21 gegen das untere Ende 14 des Verbindungsteils 5 gepresst wird. Durch den Innenkonus 24 wird das aussen komplementär konische Zwischenelement 30 radial komprimiert und das Kopfsegment 4 des Knochenfixationsmittels 1 im Hohlraum 33 des Zwischenelementes 30 blockiert. Auch hier ist die Vorrichtung in ihrem nicht blockierten Zustand dargestellt. Das Zwischenelement 30 kann verspannt werden bis die Verspannungsdistanz S überwunden ist und das Zwischenstück 21 auf dem Absatz 19 am unteren Ende 14 des Verbindungsteils 5 aufliegt.

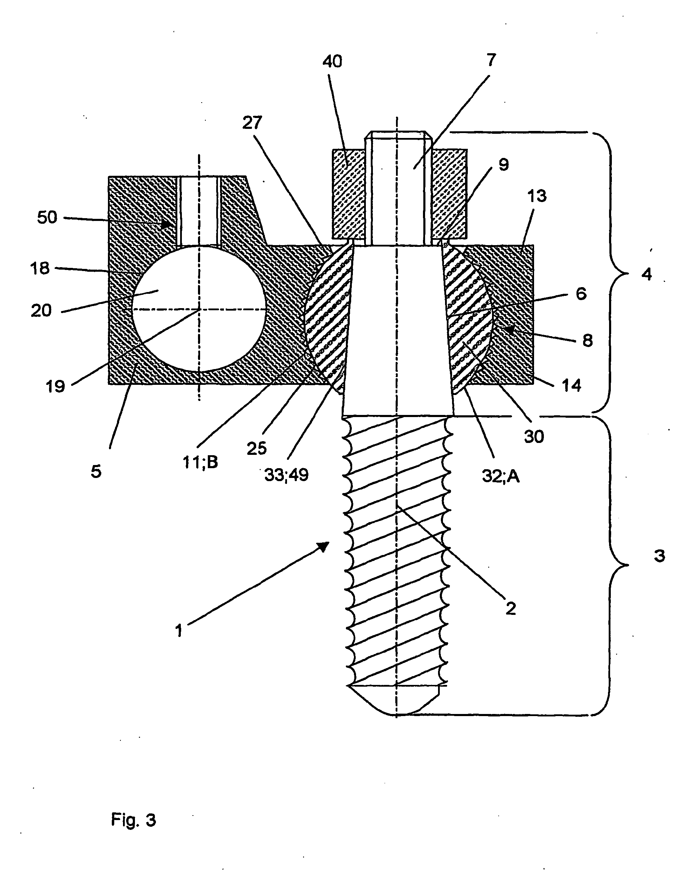

In Fig. 3 ist eine Ausführungsform dargestellt, bei welcher die Fixierung des Längsträgers 20 und die Blockierung des Kopfsegmentes 4 des Knochenfixationsmittels 1 unabhängig voneinander erfolgen. Das Kugelgelenk 8 zwischen dem Verbindungsstück 5 und dem Knochenfixationsmittel 1 wird hier durch das Verbindungsteil 5 und das Zwischenelement 30 gebildet, wobei die Wand 27 der Kavität 11 die konkave Kontaktzone B bildet und die Aussenwand 32 des Zwischenelementes 30 die konvexe Kontaktzone A bildet. Das Verbindungsteil 5 umfasst einen die Kavität 11 nicht schneidenden Kanal 18 und ein zur Fixierung des Längsträgers 20 im Kanal quer zur Kanalachse 19 einschraubbares Fixationsmittel 50, welches hier als Spannschraube ausgebildet ist. Der Kanal 18 durchdringt das Verbindungsteil 5 auch hier quer zur Zentralachse 2 des Knochenfixationselementes 1 und ist auf seinem Umfang geschlossen. Neben dem Kanal 18 ist die sphärisch ausgebildete Kavität 11 angeordnet. Die makroskopische Strukturierung 25 ist hier an der Wand 27 der Kavität 11 angebracht. Das Zwischenelement 30 ist aussen zur Kavität 11 komplementär sphärisch ausgebildet, während der Hohlraum 33 des Zwischenelementes 30 als konische Zentralbohrung 49 ausgebildet ist, welche sich vom oberen Ende 13 des Verbindungsteils 5 zum unteren Ende 14 des Verbindungsteils 5 erweitert. Das Kopfsegment 4 des Knochenfixationsmittels 1 umfasst hier einen zur konischen Zentralbohrung 49 komplementären Konus 6 und endständig einen Gewindeschaft 6, worüber die hier als Mutter ausgebildeten Spannmittel 40 schraubbar sind. Die Fixierung des Längsträgers 20 im Kanal 18 erfolgt unabhängig von der Bedienung der Spannmittel 40 mittels der Fixationsmittel 50. Auch hier ist die Vorrichtung in ihrem nicht blockierten Zustand dargestellt. Das Zwischenelement 30 kann verspannt werden bis die Verspannungsdistanz S überwunden ist und das Spannmittel 40 auf der Schulter 9 zwischen dem Konus 6 und dem Gewindeschaft 7 aufliegt.

Beim Anziehen der Spannmittel 40 drücken diese beim oberen Ende 13 des Verbindungsteils 5 auf das in die Kavität 11 eingeführte Zwischenelement 30 und ziehen den Konus 5 am Kopfsegment 4 des Knochenfixationsmittels 1 in den als konische Zentralbohrung ausgebildeten Hohlraum 33 im Zwischenelement 30. Durch die Keilwirkung der in einander gepressten Koni wird das Zwischenelement 30 radial gespreizt und mit seiner Aussenwand 32 gegen die mit der makroskopischen Strukturierung 25 versehene Wand 27 der Kavität 11 gepresst. Das Material an der Aussenwand 32 des Zwischenelementes 30 beginnt während der radialen Expanison des Zwischenelementes 30 zu fliessen, so dass die Aussenwand 32 des Zwischenelementes 30 durch die makroskopische Strukturierung 25 an der Wand 27 der Kavität 11 plastisch verformt wird und zwischen der Aussenwand 32 des Zwischenelementes 30 und der Wand 27 der Kavität 11 ein Formschluss gebildet wird.

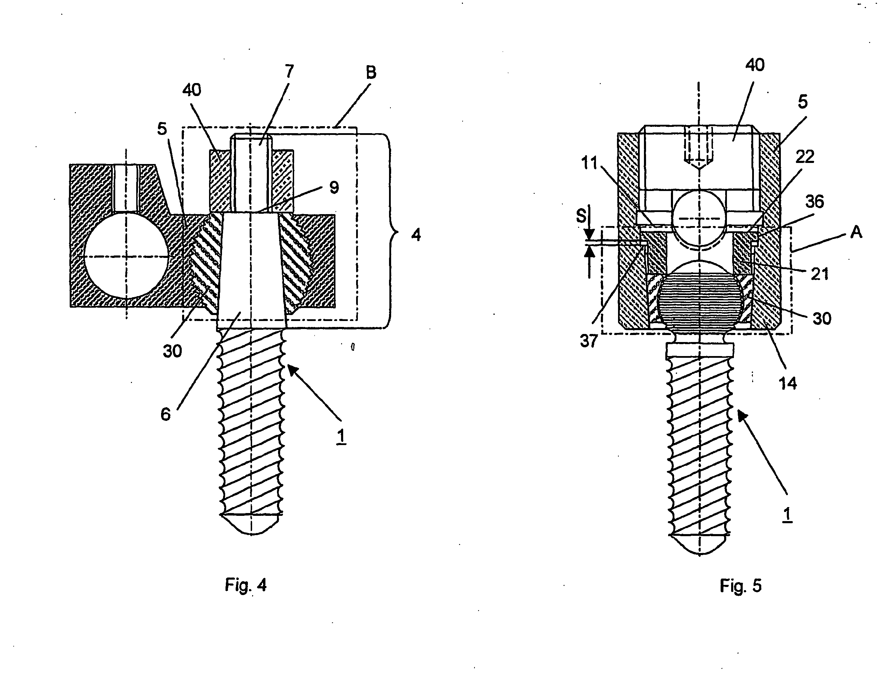

Fig. 4 zeigt die in Fig. 3 dargestellte Ausführungsform nach dem Verspannen des Spannmittels 40. Das Spannmittel 40 liegt dann auf der Schulter 9 zwischen Konus 6 und Gewindeschaft auf.

In Fig. 5 ist eine Ausführungsform dargestellt, welche sich von der in Fig. 1 dargestellten Ausführungsform nur darin unterscheidet, dass das Zwischenstück 21 an seinem oberen Ende 22 einen Bund 36 aufweist, welcher auf einer durch die Verengung zwischen dem oberen Segment 15 und der unteren Segment 16 der Kavität 11 gebildeten Auflagefläche 37 zur Anlage bringbar ist. Beim Anziehen der Spannmittel 40 wird das Zwischenstück 21 soweit gegen das untere Ende 14 des Verbindungsteils 5 gedrückt bis der Bund 36 nach dem Überwinden der Verspannungsdistanz S auf der Auflagefläche 37 aufliegt. Damit wird die Deformation des Zwischenelements 30 auf ein gewünschtes Mass begrenzt, so dass die elastische Deformierbarkeit nach der Relaxation des Zwischenelements 30 nicht eingeschränkt wird.

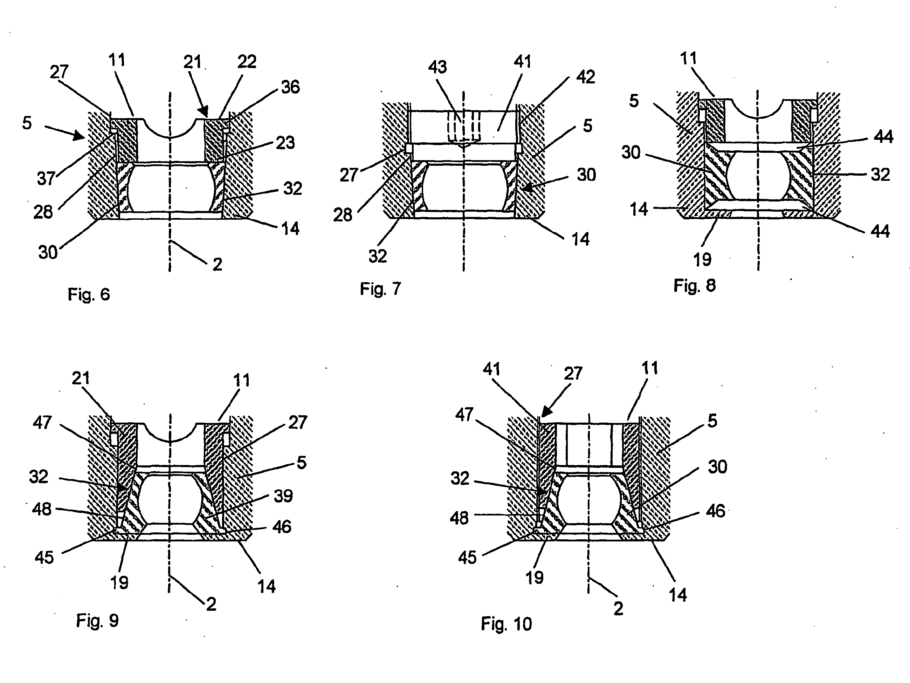

In den Fig. 6 und 7 sind Ausführungsformen der erfindungsgemässen Vorrichtung dargestellt, welche Zwischenelemente 30 enthalten, deren Aussenwände 32 gegen das untere Ende 14 des Verbindurigsteiles 5 konisch konvergieren. Die in Fig. 6 dargestellte Ausführungsform umfasst analog zu der in Fig. 5 dargestellten Ausführungsform im Verbindungsteil 5 eine Kavität 11, deren Wand 27 eine an den Innenkonus 28 angrenzende, senkrecht zur Zentralachse 2 angeordnete und konzentrische Auflagefläche 37 aufweist. An dieser Auflagefläche 37 kommt im verspannten Zustand der Vorrichtung das am oberen Ende 22 des Zwischenstückes 21 angeordnete, erweiterte Segment 36 des Zwischenstückes 21 zur Anlage. Das Zwischenstück 21 (Fig. 6) drückt beim Verspannen der Vorrichtung mittels der Spannmittel 4 (Fig. 1) mit seinem unteren Ende 23 auf das Zwischenelement 30, so dass dieses axial in den Innenkonus 28 gepresst und durch die dadurch entstehende Keilwirkung radial gegen das Kopfsegment 4 des Knochenfixationsmittels 1 (Fig. 1) gequetscht wird. Die in Fig. 7 dargestellte Ausführungsform unterscheidet sich von der in Fig. 6 dargestellten Ausführungsform nur darin, dass anstelle des axial in der Kavität 11 verschiebbaren Zwischenstückes 21 (Fig. 6) eine Spannschraube 41 vorgesehen ist, welche direkt auf das Zwischenelement 30 drückt. Die Spannschraube 41 wird in ein Innengewinde 42 eingedreht, welches in die Wand 27 der Kavität 11 an ihrem an den Innenkonus 28 angrenzenden Längsabschnitt geschnitten ist. Beim Anziehen der Spannschraube 40 drückt diese mit ihrem gegen das untere Ende 14 des Verbindungsteiles 5 gerichteten Frontseite auf das Zwischenelement 30, so dass dieses axial in den Innenkonus 28 gepresst und durch die dadurch entstehende Keilwirkung radial gegen das Kopfsegment 4 des Knochenfixationsmittels 1 (Fig. 1) gequetscht wird. Die Spannschraube 41 umfasst an ihrem gegen das obere Ende 13 des Verbindungsteiles 5 gerichteten Ende mit einer Aufnahme 43 für einen Schraubendreher versehen.

Die in Fig. 8 dargestellte Ausführungsform unterscheidet sich gegenüber der in Fig. 6 dargestellten Ausführungsformen nur darin, dass die Kavität 11 keinen Innenkonus 28 umfasst, sonder kreiszylindrisch ausgebildet ist und am unteren Ende 14 des Verbindungsteils 5 eine Verengung 19 aufweist. Das Zwischenelement 30 hat eine zur Kavität 11 komplementäre Aussenwand 32 und ist an seinen senkrecht zur Zentralachse 2 stehenden Stirnflächen mit konzentrisch konischen Vertiefungen 44 versehen. Beim Verspannen der Vorrichtung mittels der Spannmittel 40 drückt das Zwischenstück 21 mit seinem unteren Ende 23 auf die durch die konischen Vertiefungen 44 zugespitzten Stirnflächen der Aussenwand 32 des Zwischenelementes 30, so dass dieses axial und damit auch radial gegen das Kopfsegment 4 des Knochenfixationsmittels 1 (Fig. 1) gequetscht wird. Die Verspannung des Zwischenelementes 30 könnte auch analog zu der in Fig. 7 dargestellten Ausführungsform erfolgen.

Die in den Fig. 9 und 10 dargestellten Ausführungsform unterscheidet sich gegenüber den in den Fig. 6 und 7 dargestellten Ausführungsformen nur darin, dass die Kavität 11 keinen Innenkonus 28 umfasst, sonder kreizylindrisch ausgebildet ist und am unteren Ende 14 des Verbindungsteils 5 eine Verengung 19 aufweist. Zwischen der Verengung 19 und der Wand 27 der Kavität 11 ist ein Hinterstich 46 angeordnet, worin eine komplementäre Erweiterung 45 am Zwischenelement 30 eingefügt wird, so dass das Zwischenelement 30 gegen Bewegungen parallel zur Zentralachse 2 gesichert ist. Ferner verjüngt sich die Aussenwand 32 des Zwischenelementes 30 mit einem Aussenkonus 48 gegen das obere Ende 13 (Fig. 1) des Verbindungsteiles 5. Das Zwischenstück 21 (Fig. 9) oder die Spannschraube 41 (Fig.10) ist mit einem sich komplementär erweiternden Bohrungssegment 47 versehen, so dass beim Verspannen der Vorrichtung das Zwischenelement 30 das konische Bohrungssegment 47 über den Aussenkonus 48 verschoben wird und in der Folge das Zwischenelement 30 radial gequetscht wird. Die Verspannung der kugelgelenkartigen Verbindung zwischen dem Zwischenelement 30 und dem Kopfsegment 4 des Knochenfixationsmittels 1 (Fig. 1) erfolgt bei der in Fig. 9 dargestellten Ausführungsform analog zu Fig. 6 und bei der in Fig. 10 dargestellten Ausführungsform analog zur Fig. 7.

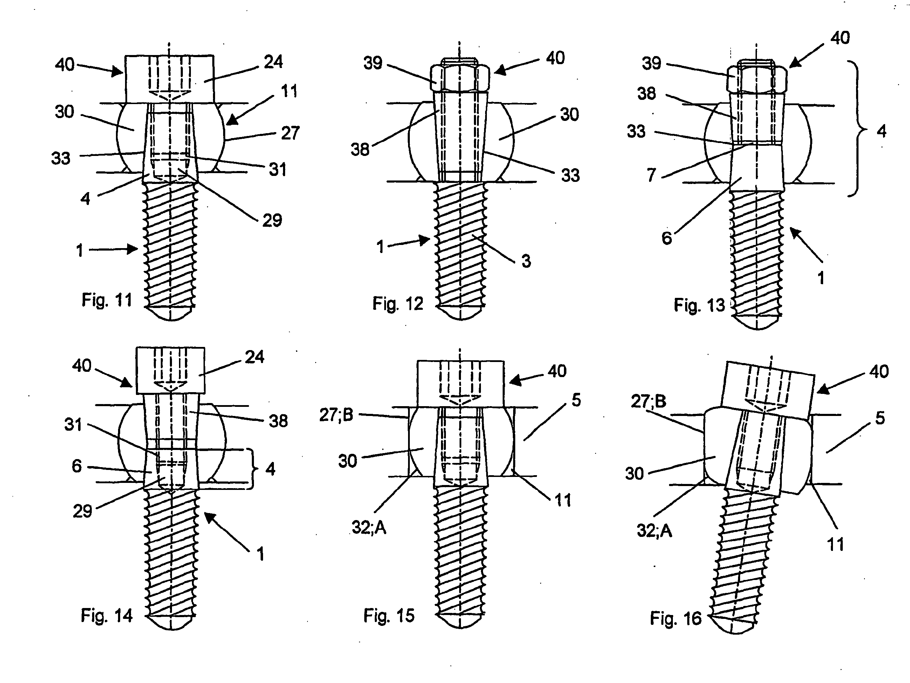

In den Fig. 11 bis 16 sind verschiedene Ausführungsformen des Zwischenelementes 30 und der Spannmittel 40 dargestellt, welche sich von der in Fig. 3 dargestellten Ausführungsform darin unterscheiden, dass
- die Spannmittel 40 durch eine Schraube 24 realisiert sind, welche in ein komplementäres, in einer endständig offenen Bohrung 29 im Kopfsegment 4 angebrachtes Innengewinde 31 schraubbar ist. Beim Anziehen der Spannmittel 40 wird analog zu der in Fig. 3 dargestellten Ausführungsform das konisch ausgebildete Kopfsegment 4 des Knochenfixationsmittels 1 in den komplementär ausgebildeten Hohlraum 33 im Zwischenelement 30 gezogen, so dass das Zwischenelement 30 expandiert und gegen die Wand 27 der Kavität 11 gepresst wird (Fig. 11);
- die Spannmittel 40 durch einen zentral durchbohrten Konus 38 und ein endständig über den Gewindeschaft 7 schraubbare Mutter 39 realisiert sind. Der zentral durchbohrte Konus 38 wird mit einem verjüngten Ende voran gegen das Verankerungssegment 3 des Knochenfixationsmittels 1 in den komplementär ausgebildeten Hohlraum 33 im Zwischenelement 30 geschoben und mittels der Mutter 39 verkeilt (Fig. 12);
- die Spannmittel 40 sich von der in Fig. 3 dargestellten Ausführungsform nur darin unterscheiden, dass der Konus 6 am Kopfsegment 4 des Knochenfixationsmittels 1 verkürzt ist und ein zentral durchbohrter Konus 38 mit seinem verjüngten Ende gegen den Konus 6 gerichtet über den Schraubenschaft 7 in den komplementär doppelt konischen Hohlraum 33 im Zwischenelement 30 geschoben wird. Mittels der als Mutter 39 ausgebildeten Spannmittel 40 werden die beiden Koni 6;38 im Hohlraum 33 verkeilt (Fig. 13);
- die in Fig. 14 dargestellten Spannmittel 40 unterscheiden sich von der in Fig. 13 dargestellten Ausführungsform nur darin, dass anstelle des Gewindeschaftes 7 (Fig. 13) am Kopfsegment 4 des Knochenfixationsmittels 1 eine koaxiale, endständig offene Bohrung 29 mit Innengewinde 31 angeordnet ist, so dass die als Schraube 24 ausgebildeten Spannmittel 40 endständig in das Kopfsegment 4 schraubbar ist, wodurch die Koni 6;38 verkeilbar sind;
- die Kavität 11 im Verbindungsteil 5 nicht zur Aussenwand 32 des Zwischenelementes 30 nicht komplementär sphärisch ausgebildet ist (Fig. 3) sondern mindestens partiell die Form eines Hohlzylinders hat (Fig. 15;16). Die Spannmittel 40 sind analog zu der in Fig. 11 dargestellten Ausführungsform ausgestaltet. Im unverspannten Zustand der Vorrichtung (Fig. 15) müssen die Wand 27 der Kavität 11 und die Aussenwand 32 des Zwischenelementes 30, welche die Kontaktzonen A;B der kugelgelenkartigen Verbindung bilden, mindestens über drei räumlich verteilte Punkte im Kontakt stehen. Beim Verspannen der Vorrichtung passt sich die im unverspannten Zustand sphärische Geometrie des Zwischenelementes 30 der asphärischen Geometrie der Wand 27 der Kavität 11 an. Im verspannten Zustand der Vorrichtung (Fig. 16) weist dann das Zwischenelement 30 eine partiell zylindrische Form auf, so dass ein Formschluss zwischen Verbindungsteil 5 und Zwischenelement 30 hergestellt wird.

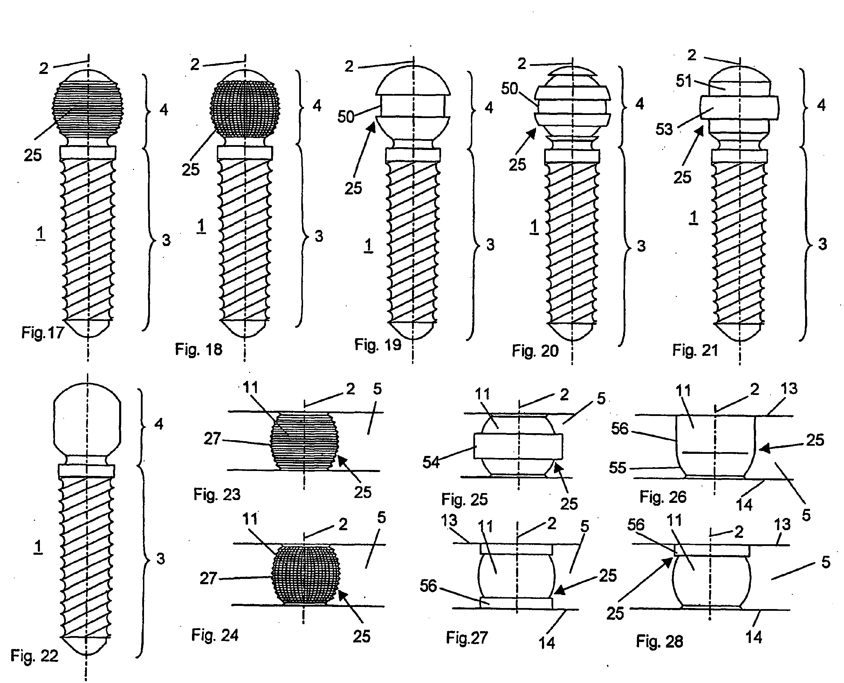

In den Fig. 17 bis 22 sind verschiedene Ausführungsformen der Knochenfixationsmittel 1 dargestellt, welche hier beispielhaft als Pedikelschrauben mit einem Schraubenschaft als Verankerungssegment 3 und einem einen im wesentlichen sphärischen Schraubenkopf als Kopfsegment 4 ausgebildet sind. Die dargestellten Knochenfixationsmittel 1 unterscheiden sich durch die Ausgestaltung der dreidimensionalen Strukturierung 25, wobei die Strukturierung 25
- in Fig. 17 als Verzahnung ausgestaltet ist;
- in Fig. 18 als Kreuzverzahnung oder Rändelung ausgestaltet ist;
- in Fig. 19 als zur Zentralachse 2 konzentrische gürtelkreisartige Nute 50 ausgebildet ist;
- in Fig. 20 drei zur Zentralachse 2 konzentrische kreisringförmige Nuten 50 umfasst;
- in Fig. 21 zwei an eine axial mittig angeordnete Kugelzone 53 axial angrenzende kreiszylindrische Segmente 51 umfasst;
- in Fig. 22 zwei diametral gegenüberliegende, seitliche Flächen 52 umfasst.

In den Fig. 23 bis 28 sind verschiedene Ausführungsformen der Kavität 11 im Verbindungsteil 5 gemäss Fig. 3 dargestellt. Die zur Zentralachse 2 konzentrische Kavität 11 weist für jede Ausführungsform eine unterschiedliche dreidimensionale Strukturierung 25 auf, wobei:
- die Kavität 11 in Fig. 23 hohlkugelzonenartig ausgebildet ist und an ihrer Wand 27 eine Verzahnung als Strukturierung 25 umfasst;
- die Kavität 11 in Fig. 24 hohlkugelzonenartig ausgebildet ist und an ihrer Wand 27 eine Kreuzverzahnung umfasst;
- die Kavität 11 in Fig. 25 hohlkugelzonenartig ausgebildet ist und einen zur Zentralachse 2 konzentrischen gürtelkreisartigen Hinterstich 54 umfasst;
- die Kavität 11 in Fig. 26 ein erstes hohlkugelzonenartiges Segment 55 und ein zweites hohlkreiszylindrisches Segment 56 umfasst, wobei das hohlkugelzonenartige Segment 55 zwischen dem unteren Ende 14 des Verbindungsteils 5 und dem zur Zentralachse 2 senkrecht stehenden Grosskreis der Kavität 11 angeordnet ist und das hohlkreiszylindrische Segment 56 zwischen dem oberen Ende 13 des Verbindungsteils 5 und dem hohlkugelzonenartigen Segment 55 angeordnet ist;
- die Kavität 11 in Fig. 27 zentral hohlkugelzonenartig ausgebildet ist und endständig je ein an das obere respektive das untere Ende 13;14 des Verbindungsteils 5 grenzendes hohlkreiszylindrisches Segment 56 umfasst; und
- die Kavität 11 in Fig. 28 sich von derjenigen in Fig. 27 nur darin unterscheidet, dass nur ein an das obere Ende 13 des Verbindungsteils 5 grenzendes hohlkreiszylindrisches Segment 56 vorgesehen ist.

## Patentansprüche

1. Vorrichtung zur dynamischen Fixierung von Knochen oder Knochenfragmenten, insbesondere von Wirbelsäulensegmenten umfassend
A) mindestens ein Knochenfixationsmittel (1) mit einer Zentralachse (2), einem an bzw. in einem Knochen verankerbaren Verankerungssegment (3) und einem axial anschliessenden Kopfsegment (4);
B) mindestens einen Längsträger (20); und
C) ein eine Längsachse (17) aufweisendes Verbindungsteil (5), welches zur lösbaren Fixation des Knochenfixationsmittels (1) am Längsträger (20) geeignet ist, mit
i. einer zur Längsachse (17) koaxialen mindestens teilweise durchgehenden Kavität (11) und einem quer zur Längsachse (17) durchgehenden Kanal (18), welcher zur Aufnahme des Längsträgers (20) geeignet ist,
ii. einem in der Kavität (11) gelagerten, elastischen Zwischenelement (30) mit einem parallel zur Längsachse (17) mindestens teilweise durchgehenden, eine Hohlraumwand (34) aufweisenden Hohlraum (33) zur Aufnahme des Kopfsegmentes (4) des Knochenfixationsmittels (1) und einer Außenwand (32); und
iii. einem Spannmittel (40), mittels welchem das Zwischenelement (30) direkt oder indirekt radial deformierbar ist, wodurch das Kopfsegment (4) im Hohlraum (33) verspannbar ist, wobei
D) die Kavität (11) im Verbindungsteil (5) zur Außenwand (32) mindestens teilweise die Form eines Hohlzylinders hat,
**dadurch gekennzeichnet, dass**
E) das Zwischenelement (30) und das Verbindungsteil (5) miteinander in Berührung stehende Kontaktzonen A;B aufweisen, die im unverspannten Zustand eine kugelgelenkartige Bewegung zwischen dem Verbindungsteil (5) und dem Knochenfixationsmittel (1) zulassen und im verspannten Zustand einen spannungsarmen, flexiblen Formschluss bilden;
F) die Außenwand (32) des Zwischenelements (30) zumindest im die kugelgelenkartige Bewegung definierenden Bereich im unverspannten Zustand mindestens partiell sphärisch konvex ausgebildet ist,
G) die Kavität (11) eine Wand (27) hat, wobei im unverspannten Zustand die Wand (27) der Kavität (11) und die Aussenwand (32) des Zwischenelementes (30), welche die Kontaktzonen A;B der kugelgelenkartigen Verbindung bilden, mindestens über drei räumlich verteilte Punkte im Kontakt stehen; und
H) das Zwischenelement (30) mindestens teilweise aus einem visko-elastischen Material besteht.

2. Vorrichtung nach Anspruch 1, **dadurch gekennzeichnet, dass** die zwei im unverspannten Zustand eine kugelgelenkartige Verbindung bildenden Kontaktzonen A;B sich zumindest partiell auf einer Kreislinie berühren.

3. Vorrichtung nach Anspruch 1 oder 2, **dadurch gekennzeichnet, dass** die mit der am Zwischenelement (30) angeordneten Kontaktzone A in Berührung stehende Kontaktzone B zumindest im Kontaktbereich verzahnt ist.

4. Vorrichtung nach Anspruch 1 oder 2, **dadurch gekennzeichnet, dass** die mit der am Zwischenelement (30) angeordneten Kontaktzone A in Berührung stehende Kontaktzone B zumindest im Kontaktbereich asphärisch ausgebildet ist.

5. Vorrichtung nach Anspruch 1 oder 2, **dadurch gekennzeichnet, dass** die mit der am Zwischenelement (30) angeordneten Kontaktzone A in Berührung stehende Kontaktzone B im Kontaktbereich rotationssymmetrisch ausgebildet ist.

6. Vorrichtung nach Anspruch 1 oder 2, **dadurch gekennzeichnet, dass** die mit der am Zwischenelement (30) angeordneten Kontaktzone A in Berührung stehende Kontaktzone B zumindest im Kontaktbereich zylindrisch ausgebildet ist.

7. Vorrichtung nach Anspruch 6, **dadurch gekennzeichnet, dass** das Zwischenelement (30) eine sphärische Aussenwand (32) aufweist und die Kavität (11) als zur Zentralachse (2) koaxial Bohrung ausgebildet ist.

8. Vorrichtung nach Anspruch 7, **dadurch gekennzeichnet, dass** im Bereich der Kavität (11) zwischen dem Zwischenelement (30) und dem Verbindungsteil (5) ein Anschlag vorgesehen ist, mittels welche die Bewegung des Zwischenelementes (30) relativ zum Verbindungsteil (5) eingeschränkt wird.

9. Vorrichtung nach Anspruch 1 oder 2, **dadurch gekennzeichnet, dass** die mit der am Zwischenelement (30) angeordneten Kontaktzone A in Berührung stehende Kontaktzone B im Kontaktbereich mindestens eine Nute (50) aufweist.

10. Vorrichtung nach Anspruch 1 oder 2, **dadurch gekennzeichnet, dass** die mit der am Zwischenelement (30) angeordneten Kontaktzone B in Berührung stehende Kontaktzone B im Kontaktbereich eine Kugelzone (53) mit mindestens einem axial endständigen kreiszylindrischen Segment (51) oder im Kontaktbereich ein hohlkugelartiges Segment (55) mit mindestens einem axial endständigen hohlkreiszylindrischen Segment (56) aufweist.

11. Vorrichtung nach einem der Ansprüche 1 bis 10, **dadurch gekennzeichnet, dass** das Zwischenelement (30) mindestens teilweise aus einem superelastischen Material besteht.

12. Vorrichtung nach Anspruch 1, **dadurch gekennzeichnet, dass** das viskoelastische Verhalten des viskoelastischen Materials durch ein Voigt-Modell angenähert wird.

13. Vorrichtung nach einem der Ansprüche 1 bis 10, **dadurch gekennzeichnet, dass** das Zwischenelement (30) mindestens teilweise aus einem elasto-viskoelastischen Material besteht.

14. Vorrichtung nach Anspruch 13, **dadurch gekennzeichnet, dass** das elastoviskoelastische Verhalten des elasto-viskoelastischen Materials durch ein Voigt-Modell mit seriell angefügter Feder angenähert wird.

15. Vorrichtung nach Anspruch 1 oder 12, **dadurch gekennzeichnet, dass** das viskoelastische Material eine Shore-Härte zwischen 50A und 90A aufweist.

16. Vorrichtung nach Anspruch 1 oder 12, **dadurch gekennzeichnet, dass** das viskoelastische Material eine Shore-Härte zwischen 50D und 90D aufweist.

17. Vorrichtung nach Anspruch 11, **dadurch gekennzeichnet, dass** das superelastische Material Nitinol ist.

18. Vorrichtung nach Anspruch 11 oder 17, **dadurch gekennzeichnet, dass** das superelastische Material einen E-Modul zwischen 20 GPa und 50 GPa aufweist.

19. Vorrichtung nach Anspruch 11 oder 17, **dadurch gekennzeichnet, dass** das superelastische Material einen E-Modul zwischen 50 GPa und 90 GPa aufweist.

20. Vorrichtung nach einem der Ansprüche 1 bis 19, **dadurch gekennzeichnet, dass** das Material des Zwischenelementes (30) sich bei zyklischer Belastung über 0,1 mHz, vorzugsweise über 1 mHz vorwiegend elastisch verhält.

21. Vorrichtung nach einem der Ansprüche 1 bis 19, **dadurch gekennzeichnet, dass** das Material des Zwischenelementes (30) sich bei zyklischer Belastung über 0,01 Hz, vorzugsweise über 0,1 Hz vorwiegend elastisch verhält.

22. Vorrichtung nach einem der Ansprüche 1 bis 19, **dadurch gekennzeichnet, dass** das Material des Zwischenelementes (30) sich bei zyklischer Belastung über 0,1 Hz, vorzugsweise über 1 Hz vorwiegend elastisch verhält.

23. Vorrichtung nach einem der Ansprüche 1 bis 22, **dadurch gekennzeichnet, dass** der Hohlraum (33) konkav ausgebildet ist, und zur kugelgelenkartigen Aufnahme des Kopfsegmentes (4) des Knochenfixationsmittels (1) geeignet ist.

24. Vorrichtung nach einem der Ansprüche 1 bis 23, **dadurch gekennzeichnet, dass** das Kopfsegment (4) konvex ausgebildet ist.

25. Vorrichtung nach einem der Ansprüche 1 bis 22, **dadurch gekennzeichnet, dass** die Kavität (11) konkav ausgebildet ist, und zur kugelgelenkartigen Aufnahme des Zwischenelementes (30) geeignet ist.

26. Vorrichtung nach Anspruch 25, **dadurch gekennzeichnet, dass** das Zwischenelement (30) eine konvexe Aussenwand (32) aufweist.

27. Vorrichtung nach einem der Ansprüche 1 bis 26, **dadurch gekennzeichnet, dass** die makroskopische Strukturierung (25) durch Erhebungen ausgestaltet sind, welche eine Höhe zwischen 0,5 mm und 1,5 mm, vorzugsweise zwischen 0,8 mm und 1,2 mm aufweisen.

28. Vorrichtung nach einem der Ansprüche 1 bis 27, **dadurch gekennzeichnet, dass** die Strukturierung (25) durch eine Verzahnung realisiert ist.

29. Vorrichtung nach einem der Ansprüche 1 bis 27, **dadurch gekennzeichnet, dass** die Strukturierung (25) durch pyramiden- oder kegelförmige Erhebungen realisiert ist.

30. Vorrichtung nach einem der Ansprüche 1 bis 29, **dadurch gekennzeichnet, dass** mindestens eine Wand des Zwischenelementes (30) mit einer Schicht aus einem hochelastischen, biokompatiblen Kunststoff versehen ist.

31. Vorrichtung nach Anspruch 30, **dadurch gekennzeichnet, dass** die Kunststoffschicht auf die Oberfläche des Zwischenelementes (30) aufgespritzt ist.

32. Vorrichtung nach Anspruch 30 oder 31, **dadurch gekennzeichnet, dass** die Dicke der Schicht zwischen 2 mm und 5 mm, vorzugsweise zwischen 3 mm und 4 mm beträgt.

33. Vorrichtung nach einem der Ansprüche 1 bis 32, **dadurch gekennzeichnet, dass** das Zwischenelement (30) aus Polyurethan, Polycarbonaturethan oder PEEK besteht.

34. Vorrichtung nach einem der Ansprüche 1 bis 33, **dadurch gekennzeichnet, dass** der Weg des Spannmittels (40) parallel zur Längsachse (17) mechanisch auf eine Verspannungsdistanz S begrenzt ist.

## Claims

1. A device for the dynamic fixation of bones or bone fragments, in particular of spinal column segments, comprising
A) at least one bone fixation device (1) with a central axis (2), an anchoring segment (3) capable of being anchored to a bone, and an axially adjoining head segment (4);
B) at least one longitudinal support (20); and
C) a connecting element (5) with a longitudinal axis (17), suitable for a releasable fixation of the bone fixation device (1) to the longitudinal support (20), with
i. an at least partially continuous cavity (11) coaxial with the longitudinal axis (17) and a through channel (18) transverse to the longitudinal axis (17), suitable for receiving the longitudinal support (20);
ii. an elastic intermediate element (30) housed in the cavity (11), with an at least partially continuous hollow space (33) parallel to the longitudinal axis (17) having a hollow space wall (34) to receive the head segment (4) of the bone fixation device (1) and an outer wall (32); and
iii. a locking means (40) by which means the intermediate element (30) can be radially deformed directly or indirectly, so that the head segment (4) can be fastened in the hollow space (33), wherein
D) the cavity (11) in the connection element (5) to the outer wall (32) has at least partially the shape of a hollow cylinder,
**characterized in that**
E) the intermediate element (30) and the connecting element (5) present contact zones A;B in contact with each other which, in the unfastened state, permit a ball-and-socket joint like movement between the connecting element (5) and the bone fixation device (1) and, in the fastened state, form a flexible, low stress positive fit;
F) the outer wall (32) of the intermediate element (30), at least in the region defining the ball-and-socket joint like movement, is configured at least partially spherically convex in the unfastened state;
G) the cavity (11) defines a wall (27) and wherein in the unfastened state the wall (27) of the cavity (11) and the outer wall (32) of the intermediate element (30), which form the contact zones A;B of the ball-and-socket joint like connection, are in contact at least at three points distributed in space; and
H) the intermediate element (30) consists at least partially of a visco-elastic material.

2. Device according to claim 1, **characterized in that** the two contact zones A;B, which form a ball-and-socket type connection in an untightened condition, contact each other at least partially on a circular line.

3. Device according to claim 1 or 2, **characterized in that** the contact zone B, which is set in contact with the contact zone A arranged at the intermediate element (30), is at least in the contact zone conformed in an indented manner.

4. Device according to claim 1 or 2, **characterized in that** the contact zone B, which is set in contact with the contact zone A arranged at the intermediate element (30), is at least in the contact zone conformed in a non-spherical manner.

5. Device according to claim 1 or 2, **characterized in that** the contact zone B, which is set in contact with the contact zone A arranged at the intermediate element (30), is at least in the contact zone conformed in a rotationally symmetrical manner.

6. Device according to claim 1 or 2, **characterized in that** the contact zone B, which is set in contact with the contact zone A arranged at the intermediate element (30), is cylindrically configured in the contact zone.

7. Device according to claim 6, **characterized in that** the intermediate element (30) presents a spherical outer wall (32) and that the cavity (11) is conformed as a borehole coaxial to the central axis (2).

8. Device according to claim 7, **characterized in that** it provides in the area of the cavity (11) between the intermediate element (30) and the connecting element (5) a shoulder which limits the motion of the intermediate element (30) with respect to the connecting element (5).

9. Device according to claim 1 or 2, **characterized in that** the contact zone B, which is in contact with the contact zone A arranged on the intermediate element (30), presents in the contact area at least one groove (50).

10. Device according to claim 1 or 2, **characterized in that** the contact zone B, which is in contact with the contact zone A arranged at the intermediate element (30), presents in the contact zone a spherical zone (53) with at least one circular cylindrical segment axially set at an extremity, or in the contact zone a hollow sphere-like segment (55) with at least one hollow circular cylindrical segment (56) axially set at an extremity.

11. Device according to one of the claims from 1 to 10, **characterized in that** the intermediate element (30) consists at least partially of a super-elastic material.

12. Device according to claim 1, **characterized in that** the visco-elastic behaviour of the visco-elastic material is approximated by a Voigt model.

13. Device according to one of the claims from 1 to 10, **characterized in that** the intermediate element (30) consists at least partially of an elasto-visco-elastic material.

14. Device according to claim 13, **characterized in that** the elasto-visco-elastic behaviour of the elasto-visco-elastic material is approximated by a Voigt model connected in series with a spring.

15. Device according to claim 1 or 12, **characterized in that** the visco-elastic material has a Shore Hardness between 50A and 90A.

16. Device according to claim 1 or 12, **characterized in that** the visco-elastic material has a Shore Hardness between 50D and 90D.

17. Device according to claim 11, **characterized in that** the super-elastic material is Nitinol.

18. Device according to claim 11 or 17, **characterized in that** the super-elastic material has an E-module between 20 GPa and 50 GPa.

19. Device according to claim 11 or 17, **characterized in that** the super-elastic material has an E-module between 50 GPa and 90 GPa.

20. Device according to one of the claims from 1 to 19, **characterized in that** the material of the intermediate element (30) behaves in a prevalently elastic manner at a cyclical loading of over 0.1 mHz, preferably over 1 mHz.

21. Device according to one of the claims from 1 to 19, **characterized in that** the material of the intermediate element (30) behaves in a prevalently elastic manner at a cyclical loading of over 0.01 Hz, preferably over 0.1 Hz.

22. Device according to one of the claims from 1 to 19, **characterized in that** the material of the intermediate element (30) behaves in a prevalently elastic manner at a cyclical loading of over 0.1 Hz, preferably over 1 Hz.

23. Device according to one of the claims from 1 to 22, **characterized in that** the hollow space (33) is conformed in a concave manner, and suitable for a ball-and-socket type acceptance of the head segment (4) of the bone fixation device (1).

24. Device according to one of the claims from 1 to 23, **characterized in that** the head segment (4) is conformed in a convex manner.

25. Device according to one of the claims from 1 to 22, **characterized in that** the cavity (11) is conformed in a concave manner and suitable for a ball-and-socket type acceptance of the intermediate element (30).

26. Device according to claim 25, **characterized in that** the intermediate element (30) presents a convex outer wall (32).

27. Device according to one of the claims from 1 to 26, **characterized in that** the macroscopic structure (25) is configured with elevations of a height between 0.5 mm and 1.5 mm, preferably between 0.8 mm and 1.2 mm.

28. Device according to one of the claims from 1 to 27, **characterized in that** the structure (25) is realized by indentations.

29. Device according to one of the claims from 1 to 27, **characterized in that** the structure (25) is realized by pyramidal or cone-shaped elevations.

30. Device according to one of the claims from 1 to 29, **characterized in that** at least one wall of the intermediate element (30) is provided with a layer made of a highly elastic, biocompatible synthetic material.

31. Device according to claim 30, **characterized in that** the layer of synthetic material is sprayed on the surface of the intermediate element (30).

32. Device according to claim 30 or 31, **characterized in that** the thickness of the layer is between 2 mm and 5 mm, preferably between 3 mm and 4 mm.

33. Device according to one of the claims from 1 to 32, **characterized in that** the intermediate element (30) consists of polyurethane, polycarbonate urethane or PEEK.

34. Device according to one of the claims from 1 to 33, **characterized in that** the path of the locking means (40) is mechanically limited, in a direction parallel to the longitudinal axis (17), to a tightening distance S.

## Revendications

1. Dispositif de fixation dynamique d'os ou de fragments d'os, en particulier de segments de colonne vertébrale, comprenant
A) au moins un moyen de fixation d'os (1) avec un axe central (2), un segment d'ancrage (3) pouvant être ancré sur ou dans un os, et un segment de tête (4) se raccordant axialement ;
B) au moins un longeron (20) ; et
C) une pièce de raccordement (5) présentant un axe longitudinal (17) et qui est appropriée pour la fixation amovible du moyen de fixation d'os (1) sur le longeron (20), avec
i. une cavité (11) au moins partiellement continue coaxiale à l'axe longitudinal (17) et un canal (18) continu transversalement à l'axe longitudinal (17) et qui est approprié pour loger le longeron (20),
ii. un élément intermédiaire (30) élastique, supporté dans la cavité (11), avec un espace creux (33), au moins partiellement continu parallèlement à l'axe longitudinal (17) et présentant une paroi d'espace creux (34), pour le logement du segment de tête (4) du moyen de fixation d'os (1), et une paroi extérieure (32) et
iii. un moyen de serrage (40) au moyen duquel l'élément intermédiaire (30) est déformable radialement directement ou indirectement, ce qui fait que le segment de tête (4) peut être serré dans l'espace creux (33),
D) la cavité (11) dans la pièce de raccordement (5) vers la paroi extérieure (32) ayant au moins partiellement la forme d'un cylindre creux,
**caractérisé en ce que**
E) l'élément intermédiaire (30) et la pièce de raccordement (5) présentent des zones de contact A;B en contact l'une avec l'autre et qui, dans l'état non serré, permettent un mouvement du type articulation à rotule entre la pièce de raccordement (5) et le moyen de fixation d'os (1), et, dans l'état serré, forment une liaison de forme flexible pauvre en tension ;
F) la paroi extérieure (32) de l'élément intermédiaire (30) est, au moins dans la zone définissant le mouvement du type articulation à rotule, dans l'état non serré, constituée de façon au moins partiellement convexe et sphérique,
G) la cavité (11) a une paroi (27) ; la paroi (27) de la cavité (11) et la paroi extérieure (32) de l'élément intermédiaire (30), qui forment les zones de contact A;B du raccordement du type articulation à rotule, étant, dans l'état non serré, en contact au moins par le biais de trois points répartis dans l'espace ; et
H) l'élément intermédiaire (30) se compose au moins partiellement d'un matériau viscoélastique.

2. Dispositif selon la revendication 1, **caractérisé en ce que** les deux zones de contact A;B formant, dans l'état non serré, un raccordement du type articulation à rotule, se touchent au moins partiellement sur une ligne circulaire.

3. Dispositif selon la revendication 1 ou 2, **caractérisé en ce que** la zone de contact B en contact avec la zone de contact A disposée sur l'élément intermédiaire (30) est dentée au moins dans la zone de contact.

4. Dispositif selon la revendication 1 ou 2, **caractérisé en ce que** la zone de contact B en contact avec la zone de contact A disposée sur l'élément intermédiaire (30) est constituée de façon asphérique au moins dans la zone de contact.

5. Dispositif selon la revendication 1 ou 2, **caractérisé en ce que** la zone de contact B en contact avec la zone de contact A disposée sur l'élément intermédiaire (30) est constitué avec une symétrie de rotation dans la zone de contact.

6. Dispositif selon la revendication 1 ou 2, **caractérisé en ce que** la zone de contact B en contact avec la zone de contact A disposée sur l'élément intermédiaire (30) est constituée de façon cylindrique au moins dans la zone de contact.

7. Dispositif selon la revendication 6, **caractérisé en ce que** l'élément intermédiaire (30) présente une paroi extérieure (32) sphérique, et **en ce que** la cavité (11) est constituée en tant qu'alésage coaxialement à l'axe central (2).

8. Dispositif selon la revendication 7, **caractérisé en ce que**, dans la zone de la cavité (11) entre l'élément intermédiaire (30) et la pièce de raccordement (5), il est prévu une butée au moyen de laquelle le mouvement de l'élément intermédiaire (30) relativement à la pièce de raccordement (5) est limité.

9. Dispositif selon la revendication 1 ou 2, **caractérisé en ce que** la zone de contact B en contact avec la zone de contact A disposée sur l'élément intermédiaire (30) présente au moins une rainure (50).

10. Dispositif selon la revendication 1 ou 2, **caractérisé en ce que** la zone de contact B en contact avec la zone de contact A disposée sur l'élément intermédiaire (30) présente dans la zone de contact une zone sphérique (53) avec au moins un segment (51) cylindrique circulaire en position terminale axialement, ou présente, dans la zone de contact, un segment (55) de type sphère creuse avec au moins un segment (56) cylindrique circulaire creux en position terminale axialement.

11. Dispositif selon l'une des revendications 1 à 10, **caractérisé en ce que** l'élément intermédiaire (30) se compose au moins partiellement d'un matériau super élastique.

12. Dispositif selon la revendication 1, **caractérisé en ce que** le comportement viscoélastique du matériau viscoélastique est approché à l'aide d'un modèle de Voigt.

13. Dispositif selon l'une des revendications 1 à 10, **caractérisé en ce que** l'élément intermédiaire (30) se compose au moins partiellement d'un matériau élasto-viscoélastique.

14. Dispositif selon la revendication 13, **caractérisé en ce que** le comportement élasto-viscoélastique du matériau élasto-viscoélastique est approché à l'aide d'un modèle de Voigt avec ressort ajouté en série.

15. Dispositif selon la revendication 1 ou 12, **caractérisé en ce que** le matériau viscoélastique présente une dureté Shore entre 50A et 90A.

16. Dispositif selon la revendication 1 ou 12, **caractérisé en ce que** le matériau viscoélastique présente une dureté Shore entre 50D et 90D.

17. Dispositif selon la revendication 11, **caractérisé en ce que** le matériau super élastique est du Nitinol.

18. Dispositif selon la revendication 11 ou 17, **caractérisé en ce que** le matériau super élastique présente un module E entre 20 GPa et 50 GPa.

19. Dispositif selon la revendication 11 ou 17, **caractérisé en ce que** le matériau super élastique présente un module E entre 50 GPa et 90 GPa.

20. Dispositif selon l'une des revendications 1 à 19, **caractérisé en ce que** le matériau de l'élément intermédiaire (30) se comporte de façon principalement élastique pour une contrainte cyclique supérieure à 0,1 mHz, de préférence supérieure à 1 mHz.

21. Dispositif selon l'une des revendications 1 à 19, **caractérisé en ce que** le matériau de l'élément intermédiaire (30) se comporte de façon principalement élastique pour une contrainte cyclique supérieure à 0,01 Hz, de préférence supérieure à 0,1 Hz.

22. Dispositif selon l'une des revendications 1 à 19, **caractérisé en ce que** le matériau de l'élément intermédiaire (30) se comporte de façon principalement élastique pour une contrainte cyclique supérieure à 0,1 Hz, de préférence supérieure à 1 Hz.

23. Dispositif selon l'une des revendications 1 à 22, **caractérisé en ce que** l'espace creux (33) est constitué de façon concave, et est approprié pour loger à la façon d'une articulation à rotule le segment de tête (4) du moyen de fixation d'os (1).

24. Dispositif selon l'une des revendications 1 à 23, **caractérisé en ce que** le segment de tête (4) est constitué de façon convexe.

25. Dispositif selon l'une des revendications 1 à 22, **caractérisé en ce que** la cavité (11) est constituée de façon concave, et est appropriée pour loger à la façon d'une articulation à rotule l'élément intermédiaire (30).

26. Dispositif selon la revendication 25, **caractérisé en ce que** l'élément intermédiaire (30) présente une paroi extérieure (32) convexe.

27. Dispositif selon l'une des revendications 1 à 26, **caractérisé en ce que** la structuration (25) macroscopique est constituée par des surélévations qui présentent une hauteur entre 0,5 mm et 1,5 mm, de préférence entre 0,8 mm et 1,2 mm.

28. Dispositif selon l'une des revendications 1 à 27, **caractérisé en ce que** la structuration (25) est réalisée par une denture.

29. Dispositif selon l'une des revendications 1 à 27, **caractérisé en ce que** la structuration (25) est réalisée par des surélévations pyramidales ou coniques.

30. Dispositif selon l'une des revendications 1 à 29, **caractérisé en ce qu'**au moins une paroi de l'élément intermédiaire (30) est munie d'une couche en matière plastique fortement élastique et biocompatible.

31. Dispositif selon la revendication 30, **caractérisé en ce que** la couche de matière plastique est appliquée au pistolet sur la surface de l'élément intermédiaire (30).

32. Dispositif selon la revendication 30 ou 31, **caractérisé en ce que** l'épaisseur de la couche est comprise entre 2 mm et 5 mm, de préférence entre 3 mm et 4 mm.

33. Dispositif selon l'une des revendications 1 à 32, **caractérisé en ce que** l'élément intermédiaire (30) est composé de polyuréthane, en polycarbonate uréthane ou en PEEK.

34. Dispositif selon l'une des revendications 1 à 33, **caractérisé en ce que** la course du moyen de serrage (40) parallèlement à l'axe longitudinal (17) est limitée mécaniquement à une distance de contraction S.
